Europäisches Patentamt

European Patent Office

Office européen des brevets

(11) Publication number: **0 156 160**
**B1**

## EUROPEAN PATENT SPECIFICATION

(45) Date of publication of patent specification: **31.01.90**

(51) Int. Cl.⁵: **A 61 L 15/00, D 04 H 1/56**

(21) Application number: **85101824.2**

(22) Date of filing: **24.11.82**

(60) Publication number of the earlier application in accordance with Art. 76 EPC: **0 080 382**

(54) Microfibre web product.

(30) Priority: **24.11.81 GB 8135331**

(43) Date of publication of application:
**02.10.85 Bulletin 85/40**

(45) Publication of the grant of the patent:
**31.01.90 Bulletin 90/05**

(84) Designated Contracting States:
**AT BE CH DE FR IT LI LU NL**

(56) References cited:
**FR-A-1 527 536**
**US-A-2 464 301**
**US-A-2 988 469**
**US-A-3 016 599**
**US-A-3 947 537**
**US-A-4 100 324**
**US-A-4 103 058**
**US-A-4 186 165**

(73) Proprietor: **KIMBERLY-CLARK LIMITED**
**Larkfield**
**Maidstone, Kent, ME20 7PS (GB)**

(72) Inventor: **Minto, Mansoor Ahmad, Dr.**
**2 Jerome Road**
**Larkfield Kent (GB)**
Inventor: **Storey, Dennis Graham**
**1a The Landway**
**Bearsted Kent (GB)**
Inventor: **Owen, Geoffrey Robert, Dr.**
**55 Cherry Orchard**
**Ditton Kent (GB)**

(74) Representative: **Allen, Oliver John Richard et al**
**Lloyd Wise, Tregear & Co. Norman House**
**105-109 Strand**
**London, WC2R 0AE (GB)**

Courier Press, Leamington Spa, England.

# EP 0 156 160 B1

## Description

This invention relates to non-woven fabrics and in particular to those comprising a matrix of meltblown polymer fibres and a method of producing these..

Fabric made from meltblown polymer fibre is well known and is described, for example, in British Patent No. 2006614, British Patent No. 1295267 and U.S. Patent No. 3676242. Such a fabric will be referred to hereafter as M.B.P.F.

Fabric of meltblown polyolefin fibres is useful as wiping cloths for oil and when this is additionally treated with a wetting agent, as proposed in British Patent No. 2006614 it has excellent oil and water wiping properties. When the fabric is also treated by a pattern bonding process it is strong and durable. However, such fabric is relatively expensive when compared with disposable wipers derived from creped tissue or paper.

It is also known to treat M.B.P.F. to make it suitable for use as a filter. This is done by incorporating particles such as activated carbon or alumina without the use of binders, by intermixing with the fibres. The particles are retained by mechanical entanglement with the fibres and do not adhere to the microfibre. Such material is unsuitable for use as a wiper since the particles are not sufficiently well retained and would tend to "dust out" or drop out of the material if used as a wiper.

US Patent No. 4 100 324 describes a method of making a fluid retentive non-woven web which comprises extruding molten polymeric material in such a way as to produce a stream of molten polymeric microfibres and directing wood pulp fibres into the stream of microfibres. The wood pulp fibres are held within the microfibres, which are cooled so that they set, by mechanical entanglement of the microfibres with the wood pulp fibres. The wood pulp fibres are sufficiently large to be satisfactorily held in the web by mechanical entanglement but fibres are not as effective as particles at adsorbing a liquid.

In accordance with one aspect of the invention, a method of making a fluid retentive non-woven web comprising extruding a molten polymeric material in such a way as to produce a stream of melt blow polymeric microfibres with a diameter of between 1 and 50 $\mu$m, directing absorbent material into the stream of microfibres and cooling and quenching the fibres or otherwise allowing them to cool so that the fibres are set and then forming or consolidating the set fibres into a web characterised in that the absorbent material is particulate and in that the absorbent particles are directed into the stream of microfibres whilst the fibres are in a tacky state so that the particles adhere to be fibres, the fibres being subsequently quenched or otherwise allowed to cool so that they are set.

In accordance with another aspect of the invention, a fluid retentive web comprises meltblown thermoplastic microfibres whose diameter is between 1 and 50 $\mu$m and absorbent material characterised in that the absorbent material is particulate or granulate and in that the particles or granules are firmly adhered to the fibres by being brought into contact with the fibres whilst the fibres are still in a tacky condition.

The particles in the resulting fabric web are held firmly even if the fabric is abraded or torn when used as a wiper.

The particles are preferably blown onto the stream of particles shortly after the fibres leave an extrusion nozzle and the particles may be given an electrostatic charge prior to contacting the fibres which helps to separate the particles in the web.

The particles are suitably injected into an air stream impinging on the fibres.

Other fibres such as wood pulp fibre or staple textile fibres (e.g. cotton) may also be introduced preferably simultaneously with the absorbent particles.

Preferably the fibres of the M.B.P.F. have a diameter between 1 and 50 microns, with most fibres preferably less than 10 microns. The fibres may, for example, be of polyester, polypropylene or nylon.

A wetting agent may be added to improve the water absorbency properties. A surfactant may be added to the fibres in order to assist wetting thereof.

The particles, when the fabric is to be used, for example, as an industrial or catering wiper, may be of a wide range of low cost absorbent granular materials such as clay, kaolin, talc, calcium carbonate, sodium sulphate, sodium carbonate or aluminium oxide. It is also possible to use granular organic materials such as sponge particles. Calcined clay, particularly calcined china clay, is very useful. This has a crystalline structure and produces granules normally hollow, which are more absorbent than other clay material.

The particles may be particles of super asborbent material which are maintained in place by surface tack of the microfibres.

The particles may be relatively small, e.g. 1 micron or less up to 100 microns or larger and may be incorporated as individual particles or as cluster.

The particle size in one embodiment of the invention, using calcined china clay is 25% less than 2 microns, 28% greater than 10 microns and 3% greater than 20 microns. In this embodiment the clay was incorporated in a melt blown matrix of polypropylene at levels of approximately 6% and 14% and at a basic weight of approximately 90 g/m$^2$. It is considered that the particle size range should be between 1 and 100 microns with amounts of calcined clay of 5 to 40%. An increase of clay over 40% may tend to weaken the resultant product whilst not appreciably increasing the absorption capacity for water and/or oil.

It has been found that the clay particle additive significantly decreases the product cost by reducing the polymer content required per weight of the product.

The oil (SAE 10) absorptive capacity of the product with clay particles was found to be 1 to 2 grams of oil per gram of calcined clay.

In order to increase its strength, M.B.P.F. in accordance with the invention may be hot calendered or embossed with heated patterned bonding rolls. The fabric may also be perforated as described and claimed in EP—A—0080383. This further improves the absorbency and wiping properties of the fabric.

The invention will now be further described by way of example with reference to the accompanying drawings in which:

Figure 1 is a partly schematic side elevation of an apparatus for producing fabrics according to the present invention;

Figure 2 is a plan view of a fragment of fabric according to the present invention which has been embossed;

Figure 3 is a cross-section of one form of embossment in the fabric of Figure 2;

Figures 4 and 5 are electron microscope photographs of clay filled fabric of the present invention taken with a magnification of 5,500 and 18,000 times;

Figure 6 is an electron microscope photograph of a fabric having cellulose sponge particles; and

Figure 7 is an electron microscope photograph of fabric with super absorbent particles.

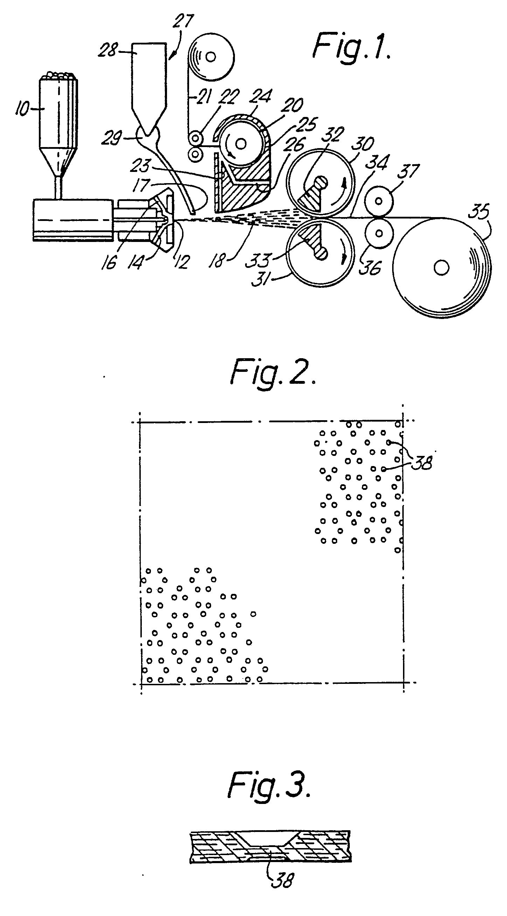

Referring to Figure 1, discontinuous thermo-plastic polymeric material from a hopper 10 is heated and then caused to flow through nozzle 12 whilst being subjected to air jets through nozzles 14, 16 which produces a final stream 18 containing discontinuous microfibres of the polymeric material. This is known as melt-blowing and the technique is further described in an article entitled "Superfine Thermoplastic Fibres" appearing in Industrial and Engineering Chemistry Vol. 48, No. 8, pp 1342—1346 which describeds work done at the Naval Research Laboratories in Washington D.C. Also see Naval Research Laboratory Report No. 11437 dated 15th April 1954, U.S. Patent No. 3,676,242 and U.S. Patent No. 4,100,32 issued to Anderson et al.

The apparatus shown in Figure 1 is generally the same as described in U.S. Patent No. 4,100,324 with the exception of two particular features which will be described hereinafter and the subject matter of that patent is to be considered as being included in the present specification and will not be further described. The subject matter of U.S. Patent No. 3,793,678 entitled "Pulp Picking Apparatus with Improved Fibre Forming Duct" is also to be considered as being included in the present specification insofar as the picker roll 20 and feed 21 to 26 are concerned, are also described in U.S. Patent No. 4,100,324.

The picker roll 20 and associated feed 21 to 26 are an optional feature of the apparatus of Figure 1 and are provided to enable the introduction of fibrous material into the web of the invention if this is required.

The picker device comprises a conventional picker roll 20 having picking teeth for divellicating pulp sheets 21 into individual fibres. The pulp sheets 21 are fed radially, i.e., along a picker roll radius, to the picker roll 20 by means of rolls 22. As the teeth on the picker roll 20 divellicate the pulp sheets 21 into individual fibres, the resulting separated fibres are conveyed downwardly toward the primary air stream through a forming nozzle or duct 23. A housing 24 encloses the picker roll 20 and provides a passage 25 between the housing 24 and the picker roll surface. Process air is supplied to the picker roll in the passage 25 via duct 26 in sufficient quantity to serve as a medium for conveying the fibres through the forming duct 23 at a velocity approaching that of the picker teeth. The air may be supplied by any conventional means as, for example, a blower.

It has been found that, in order to avoid fibre floccing, the individual fibres should be conveyed through the duct 23 at substantially the same velocity at which they leave the picker teeth after separation from the pulp sheets 21, i.e., the fibres should maintain their velocity in both magnitude and direction from the point where they leave the picker teeth. More particularly, the velocity of the fibres separated from the pulp sheets 21 preferably does not change by more than about 20% in the duct 23. This is in contrast with other forming apparatus in which, due to flow separation, fibres do not travel in an ordered manner from the picker and, consequently, fibre velocities change as much as 100% or more during conveyance.

Further details of the picker device may be found in U.S. Specification No. 4,100,324. The particular differences between the apparatus shown in Figure 1 of the present specification and that of Figure 1 of U.S. Patent No. 4,100,324 is the means 27 for introducing particulate absorbent material into the melt blown fibre stream 18. The particle introduction means comprises a hopper 28 and air impeller 29 so arranged that the particules are ejected as a stream through a nozzle 17 into the fibre mat shortly after the nozzle 12 and whilst the melt blown fibres remain unset and tacky. The particles stick to the tacky fibres and are distributed throughout the fibre mat.

The fibres then cool as they continue in their path and/or they may be quenched with an air or water jet to aid cooling so that the fibres are set, with the particles adhered to them, before the fibres are formed into a web as described hereafter.

It is also possible to introduce the absorbent particles through the picker roll 20 and nozzle 23 either as an independent stream of particles or together with a stream of wood pulp fibres or a stream of staple textile fibres.

The velocity of the air stream with which the particles are ejected onto the fibres, may be adjusted so that the majority of the particles adhere to the fibres and do not pass through the fibre stream.

The hot air forming the melt blown fibres is at similar pressures and temperatures to that disclosed in U.S. Patent No. 4,100,324.

The set fibres and particles are condensed into a web by passing the mat of fibres between rolls 30 and 31 having foraminous surfaces that rotate continuously over a pair of fixed vacuum nozzles 32 and 33. As the integrated stream 18 enters the nip of the rolls 30 and 31, the carrying gas is sucked into the two vacuum nozzles 32 and 33 while the fibre blend is supported and slightly compressed by the opposed surfaces of the two rolls 30 and 31. This forms an integrated, self-supporting fibrous web 34 that has sufficient integrity to permit it to be withdrawn from the vacuum roll nip and conveyed to a wind-up roll 35.

Alternatively, the web may be formed on a moving wire screen. The web is then further processed and bonded by hot calendering, embossing or perforating, or by ultrasonic embossing.

Heated embossing rolls 36 and 37 are provided as more fully described in EP—A—0080383. These rolls are driven at different speeds and the consolidated fibre web is passed between the rolls to emboss the web and bond it. The differential speed of the rolls causes the relatively outer fibres to be in effect lifted or "brushed up" giving an enhanced thickness to the web.

The embossments on the roll may extend further from the roll surface than the thickness of the web which also aids in achieving an enhanced web product.

Fabrics made with the apparatus shown in Figure 1 and with the apparatus shown but with the embossing head 40 and anvil roll 41 of U.S. Patent No. 4,100,324 replacing rolls 36 and 37 are shown in Figure 2, with the embossment indicated at 38 (see also Figure 3).

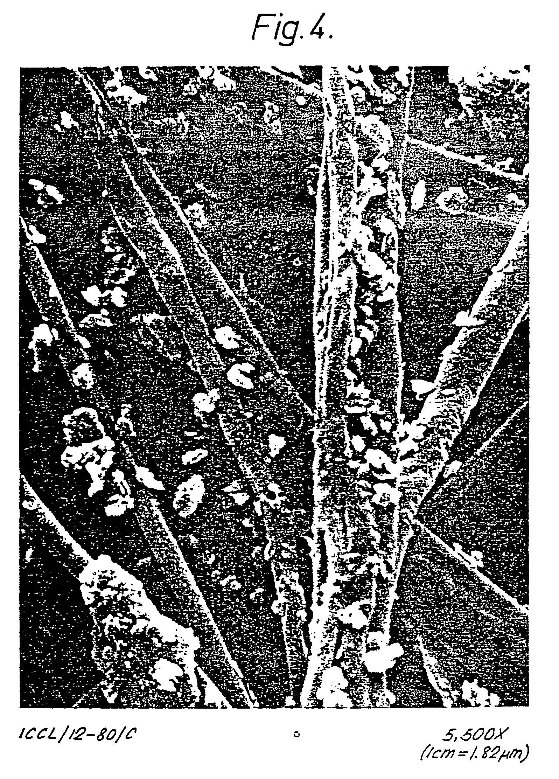

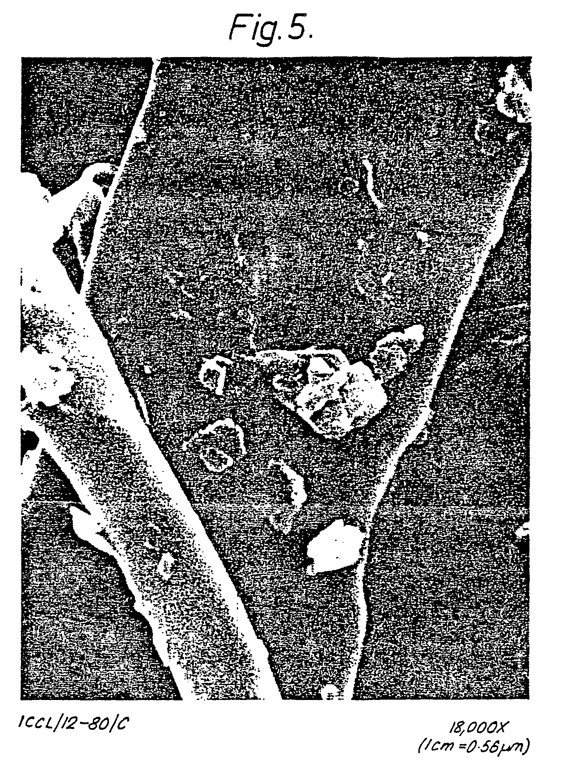

The primary feature of the invention is the inclusion of particulate material into the M.B.P.F. This is achieved by directing the particles through a nozzle into the stream of microfibres as they leave the die head, whilst the microfibres are still tacky and the particles adhere to the microfibres or even become partially embedded in them. Figures 4 to 6 clearly show that the particles are adhering to the microfibres or have become partially embedded in the fibres.

One preferred particulate material is calcined English China Clay, samples of which are listed below in Table 1.

TABLE 1

| Clay Samples No. | Source | Clay Code |
|---|---|---|
| 1 | Laporte Industries Ltd., Luton, Beds. | SKY22/44 (S) |
| 2 | BDH Chemicals Ltd. Poole, Dorset. | 33058 |
| 3 | English China Clay International, St. Austell, Cornwall. | ar-501 |
| 4 | " " " " | M-100 |
| 5 | " " " " | Superfill |
| 6 | " " " " | SPS |
| 7 | " " " " | ECR |
| 8 | " " " " | Al.BP |

Other European clays, particulary Spanish and Italian clays, may be used.

Other particulate material such as talc, calcium carbonate, sodium sulphate, kaolin, calcium sulphate, sodium carbonate, aluminium oxide or silica may be used.

Screening studies of the clays listed in Table 1 for fluid holding capacity and rate of wickability are given in Table 2 and 3 respectively. The Tables are set out at the end of this specification.

A comparative study of results in Table 2 shows that for water fluid holding capacity (gram/gram) Clay No. 1 is the best followed by Clay No. 5. The remaining clays performed reasonably well except Clay Nos. 3 and 4. The poor performance, of these could be attributed to the fact that the particle size is well below the optimal required for water, very fine particles were lost during use, and the void volume is low. The particles size in Clay Nos. 3 and 4 were generally 20% less than 1 micron, 50% less than 2 microns and 10% greater than 10 microns.

On the other hand the oil (SAE—10) holding capacity for Clay 2, 3, 4, 6 and 7 is good and the performance of the remaining three is not bad. Results in Table 2 seem to indicate that the lower range of particles in the particle size distribution appear to have a positive contribution, due mainly to increased surface area, towards the oil holding capacity of the clay.

4

Improvements in the performance of the fabric in accordance with the invention is achievable by the use of surfactants such as described in British Patent No. 2,006,614. In particular, a surfactant is applied to the microfibres in order to make the fibres wettable to aqueous solutions. It is also possible to include fibrous material as disclosed in U.S. Patent No. 4,100,324 by the means disclosed therein.

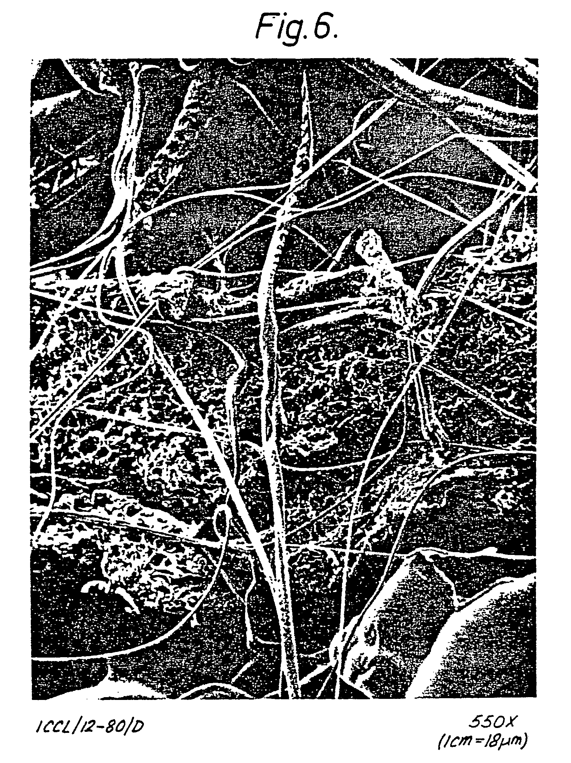

Granular organic materials may also be incorporated and particles of cellulose sponge have been used as illustrated in Figure 6. The water absorbent properties of the sponge contribute to the performance of the fabric as a water wipe. The sponge particle in Figure 6 has a dimension of about 0.16 mm in one direction.

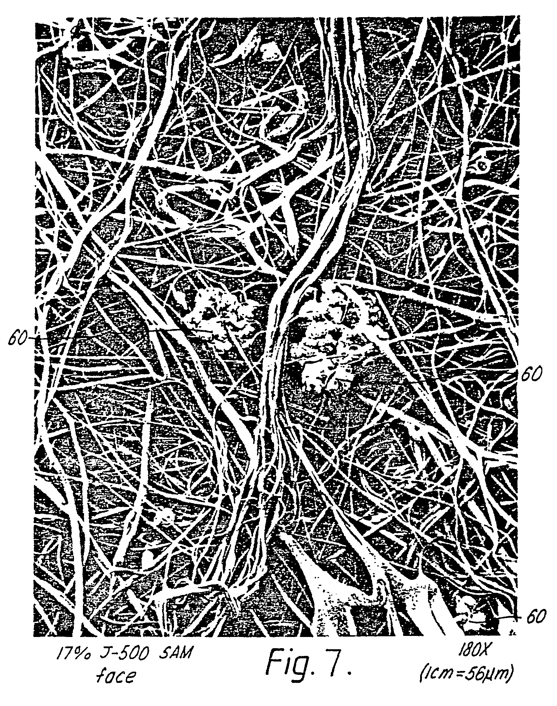

Particles in the form of particles 60 of super absorbent material may be used as illustrated in the web shown in Figure 7.

Attention is drawn to European Patent Application No. 82306269.0, out of which this divisional application is divided.

TABLE 2

Fluid (water and oil) holding capacity of clays fluid holding capacity (gram/gram)

| | For water | | For oil (SAE-10) | | |
|---|---|---|---|---|---|
| Clay No. | Atmospheric Pressure $(6.8 \times 10^3 N/m^2)$ | Approx. 3 p.s.i. $(2 \times 10^4 N/m^2)$ | Atmospheric Pressure $(6.8 \times 10^3 N/m^2)$ | Approx 3 p.s.i. $(2 \times 10^4 N/m^2)$ | Particle size distribution (microns) |
| 1 | 2.00 | 1.69 | 0.95 | 0.77 | +100 |
| 2 | 0.81 | 0.31 | 1.74 | 1.06 | ≈2—20 |
| 3 | 0.31* | 0.22* | 1.98 | | <1(20%) <2(50%) >10(10%) |
| 4 | 0.32* | | 2.01 | 1.41 | <2(15%) >10(35%) |
| 5 | 1.16 | 1.03 | 0.95 | 0.84 | |
| 6 | 0.76 | 0.37 | 1.54 | 1.58 | <2(80%) >10(0.2%) |
| 7 | 0.72 | | 2.21 | 1.39 | <(2(25%) >10(28%) |
| 8 | 0.66 | | 1.18 | 0.96 | <2(78%) >5(0.5%) |

Notes
1. The results of this table should only be considered for a relative comparison between the 8 different clays
2. * Some of the fine particles were washed down with water through and along the sides of the Whatman Filter Paper No. 1.

TABLE 3
*Rate of Wetting:* Wickability

Basis: 10 gram of material (Clay)

| Sample No. | Rate (Seconds) | Colour | Moisture Content % |
|---|---|---|---|
| 1 | 13 | Brown | 2.1 |
| 2 | 240 | Off White | 0.8 |
| 3 | 350 | Pale | 2.2 |
| 4 | 152 | White | 0.4 |
| 5 (Lumps) | 724 | Pale | 14.4 |
| 6 | 345 | White | 1.1 |
| 7 | 240 | White | 0.3 |
| 8 | 585 | Off White | 0.4 |

## Claims

1. A method of making a fluid retentive non-woven web comprising extruding a molten polymeric material in such a way as to produce a stream of melt blow polymeric microfibres with a diameter of between 1 and 50 µm, directing absorbent material into the stream of microfibres and cooling and quenching the fibres or otherwise allowing them to cool so that the fibres are set and then forming or consolidating the set fibres into a web characterised in that the absorbent material is particulate and in that the absorbent particles are directed into the stream of microfibres whilst the fibres are in a tacky state so that the particles adhere to the fibres, the fibres being subsequently quenched or otherwise allowed to cool so that they are set.

2. A method as claimed in Claim 1 in which the particles are blown onto the stream of fibres after the fibres leave an extrusion nozzle.

3. A method as claimed in either Claim 1 or 2 in which the particles are given an electrostatic charge prior to contacting the fibres.

4. A method as claimed in any one of the preceding claims in which the particles are injected into an air stream prior to the air stream impinging on the fibres.

5. A method as claimed in Claim 4 in which the velocity of the said air stream is adjusted so that the majority of the particles are adhered by melt blown fibres and do not pass through the fibre stream.

6. A method as claimed in any preceding claim in which the web is hot calendered or embossed by passing it between heated patterned bonding rolls.

7. A method as claimed in Claim 6 in which the depth of the embossing member on the patterned roll is greater than the thickness of the web.

8. A method as claimed in either Claims 6 or 7 in which the embossing rolls are driven at different speeds.

9. A method as claimed in any preceding Claim in which other fibres are introduced into the stream of microfibres prior to formulation of the fibres into a web..

10. A method as claimed in any one of the preceding claims in which a wetting agent is added to the fibres.

11. A method as claimed in any one of the preceding claims in which a surfactant is added to the fibres in order to assist wetting thereof.

12. A fluid retentive web comprising meltblown thermoplastic microfibres whose diameter is between 1 and 50 µm and absorbent material characterised in that the absorbent material is particulate or granulate and in that the particles or granules are firmly adhered to the fibres by being brought into contact with the fibres whilst the fibres are still in a tacky condition.

## Patentansprüche

1. Verfahren zur Herstellung einer Flüssigkeit zurückhaltenden Vliesbahn, bei dem man ein geschmolzenes Polymermaterial so extrudiert, daß sich ein Strom schmelzgeblasener Polymermikrofasern mit einem Durchmesser zwischen 1 und 50 μm bildet, saugfähiges Material in den Mikrofaserstrom einleitet und die Fasern kühlt und abschreckt oder sie auf andere Weise abkühlen läßt, damit die Fasern fixiert werden, und dann die fixierten Fasern zu einer Bahn formt oder verfestigt, dadurch gekennzeichnet, daß das saugfähige Material teilchenförmig ist und daß die saugfähigen Teilchen in den Mikrofaserstrom eingeleitet werden, während sich die Fasern in einem klebrigen Zustand befinden, so daß die Teilchen an den Fasern anhaften, wobei man die Fasern danach abschreckt oder auf andere Weise abkühlen läßt, damit sie fixiert werden.

2. Verfahren nach Anspruch 1, wobei die Teilchen in den Faserstrom geblasen werden, nachdem diese aus einer Extrusionsdüse austreten.

3. Verfahren nach Anspruch 1 oder 2, wobei die Teilchen mit einer elektrostatischen Ladung versehen werden, bevor sie mit den Fasern in Berührung kommen.

4. Verfahren nach einem der vorhergehenden Ansprüche, wobei die Teilchen in einen Luftstrom eingespritzt werden, bevor dieser auf die Fasern trifft.

5. Verfahren nach Anspruch 4, wobei man die Geschwindigkeit dieses Luftstroms so einstellt, daß die Mehrzahl der Teilchen an den schmelzgeblasenen Fasern anhaften und nicht durch den Faserstrom hindurchgehen.

6. Verfahren nach einem der vorhergehenden Ansprüche, wobei die Bahn heiß kalandriert oder geprägt wird, indem man sie zwischen erhitzten gemusterten Verklebungswalzen hindurchleitet.

7. Verfahren nach Anspruch 6, wobei die Tiefe des Prägeelements auf der gemusterten Walze größer als die Dicke der Bahn ist.

8. Verfahren nach Anspruch 6 oder 7, wobei die Prägewalzen mit verschiedenen Geschwindigkeiten laufen.

9. Verfahren nach einem der vorhergehenden Ansprüche, wobei andere Fasern in den Mikrofaserstrom eingeführt werden, bevor man die Fasern zu einer Bahn formt.

10. Verfahren nach einem der vorhergehenden Ansprüche, wobei den Fasern ein Netzmittel zugesetzt wird.

11. Verfahren nach einem der vorhergehenden Ansprüche, wobei den Fasern ein Tensid zugesetzt wird, um deren Benetzung zu unterstützen.

12. Flüssigkeit zurückhaltende Bahn, bestehend aus schmelzgeblasenen thermoplastischen Mikrofasern, deren Durchmesser zwischen 1 und 50 μm liegt, und saugfähigem Material, dadurch gekennzeichnet, daß das saugfähige Material teilchenförmig oder granuliert ist und daß die Teilchen oder das Granulat fest an den Fasern anhaften, indem man sie mit den Fasern in Berührung bringt, während diese sich noch in einem klebrigen Zustand befinden.

## Revendications

1. Une méthode pour fabriquer une étoffe non-tissée retenant les liquides comprenant l'extrusion d'un matériau polymère fondu de manière à produire un jet de microfibres polymères par fusion et soufflage d'un diamètre de 1 à 50 m, en dirigeant une matière absorbante dans le jet de microfibres et en refroidissant et trempant les fibres ou autrement en leur permettant de se refroidir afin que les fibres soient solidifiées et ensuite en formant ou en renforçant les fibres solidifées en une étoffe characterisée par le fait que la matière absorbante est particulaire et que les particules absorbantes sont dirigées dans le jet de microfibres pendant que les fibres sont à l'état collant si bien que les particules adhèrent aux fibres, les fibres étant ensuite trempées ou autrement laissées refroidir afin qu'elles soient solidifiées.

2. Une méthode telle que revendiquée dans la revendication 1 dans laquelle les particules sont soufflées sur le jet de fibres après que les fibres soient sorties d'une base d'extrusion.

3. Une méthode telle que revendiquée dans les revendications 1 ou 2 dans laquelle les particules reçoivent une charge électrostatique avant d'entrer en contact avec les fibres.

4. Une méthode telle que revendiquée dans une quelconque des revendications précédentes dans laquelle les particules sont injectées dans un jet d'air avant que le jet d'air ne frappe les fibres.

5. Une méthode telle que revendiquée dans la revendication 4 dans laquelle la velocite dudit jet d'air est réglée afin que la majorité des particules soient collées par les fibres fondues-soufflées et ne traversent pas le jet de fibres.

6. Une méthode telle que revendiquée dans une quelconque des revendications précédentes dans laquelle l'étoffe est calandrée à chaud ou gaufrée par passage entre des rouleaux de collage chauffés comportant des motifs.

7. Une méthode telle que revendiquée dans la revendication 6 dans laquelle la profondeur de la partie gaufrante du rouleau à motif est plus grande que l'épaisseur de l'étoffe.

8. Une méthode telle que revendiquée dans une des revendications 6 ou 7 dans laquelle les rouleaux de gaufrage sont entraînés à vitesses différentes.

9. Une méthode telle que revendiquée dans une quelconque des revendications précédentes dans laquelle d'autres fibres sont introduites dans le jet de microfibres avant que les fibres ne se forment en étoffe.

10. Une méthode telle que revendiquée dans une quelconque des revendications précédentes dans laquelle un agent mouillant est ajouté aux fibres.

11. Une méthode telle que revendiquée dans une quelconque des précédentes revendications dans laquelle un surfactant est ajouté aux fibres afin d'aider à les mouiller.

12. Une étoffe retenant les fluides comprenant des microfibres thermoplastique fondues-soufflées dont le diamètre est entre 1 et 50 m et une matière absorbante charactérisée en ce que la matière absorbante est en particules ou granulés et que les particules ou granulés sont fermement collés aux fibres par leur mise en contact avec les fibres pendant que les fibres sont encore à l'état collant.

Fig.1.

Fig.2.

Fig.3.

## Fig.4.

ICCL/12-80/C                    5,500X
                              (1cm=1.82μm)

2

## Fig.5.

ICCL/12-80/C

18,000X
(1cm = 0.56μm)

## Fig. 6.

ICCL/12-80/D

550X
(1cm = 18μm)

17% J-500 SAM
face

Fig. 7.

180X
(1cm=56μm)